# EUROPEAN PATENT SPECIFICATION

(11) **EP 2 051 706 B1**
(45) Date of publication and mention of the grant of the patent: **11.05.2016**
(21) Application number: 07801511.2
(22) Date of filing: 03.08.2007
(51) Int. Cl.: A61K 31/122, A61P 25/00, A61P 25/16, A61P 25/28, A61P 21/00

(54) **TRANSMUCOSAL ADMINISTRATION OF 2-(10-HYDROXYDECYL)-5,6-DIMETHOXY-3-METHYL-2,5-CYCLOHEXADIENE-1,4-DIONE (IDEBENONE)**
TRANSMUKOSALE VERABREICHUNG VON 2-(10-HYDROXYDECYL)-5,6-DIMETHOXY-3-METHYL-2,5-CYCLOHEXADIENE-1,4-DION (IDEBENON)
ADMINISTRATION PAR VOIE TRANSMUCOSALE DE LA 2-(10-HYDROXYDÉCYL)-5,6-DIMÉTHOXY-3-MÉTHYL-2,5-CYCLOHEXADIÈNE-1,4-DIONE (IDÉBÉNONE)

(30) Priority: 14.08.2006 EP 06016935; 14.08.2006 US 837496 P
(43) Date of publication of application: 29.04.2009
(73) Proprietor: Santhera Pharmaceuticals (Schweiz) AG, 4410 Liestal (CH)
(72) Inventor: DUBACH-POWELL, Judith, 4104 Oberwil (CH); HAUSMANN, Rudolf, 4052 Basel (CH); VANCAN, Pierre, 4125 Riehen (CH)
(74) Representative: Grünecker Patent- und Rechtsanwälte PartG mbB
(86) International application number: PCT/EP2007/006895
(87) International publication number: WO 2008/019769

(56) References cited:
- EP-A1- 1 378 753
- EP-A2- 0 553 777
- WO-A-00/25821
- WO-A-2006/007910
- WO-A-2006/100017
- US-A- 6 133 322
- US-A1- 2004 067 986
- US-B1- 6 562 363

## Description

The present invention relates to a medicament for transmucosal administration of 2,3-dimethoxy-5-methyl-6-( 10-hydroxydecyl)-1,4-benzoquinone (idebenone).

### Background of the invention:

Idebenone is a synthetic analogue of coenzyme Q10 (CoQ10), a vital cell membrane antioxidant and essential constituent of the adenosine-triphosphate (ATP) producing mitochondrial electron transport chain (ETC). Up to now, idebenone has been used in a variety of medical applications. Similar to coenzyme Q10, idebenone undergoes reduction/oxidation cycles in living organisms and reduced idebenone is an antioxidant and radical scavenger (A. Mordente, G. E. Martorana, G. Minotti, B. Giardina, Chem. Res. Toxicol. 11 (1998), 54-63). It is known that idebenone protects cell membranes and mitochondria from oxidative damage because of its ability to inhibit lipid peroxidation (M. Suno, M. Shibota, A. Nagaoka, Arch. Gerontol. Geriatr. 8 (1989), 307-311). Idebenone also interacts with the ETC, preserving ATP formation in ischemic states. It has been shown that the compound stimulates nerve growth factor, a characteristic that could be important for the treatment of Alzheimer's and other neurodegenerative diseases (K. Yamada, A. Nitta, T. Hasegawa, K. Fuji, M. Hiramatsu, T. Kameyama, Y. Furukawa, K. Hayashi, T. Nabeshima, Behav. Brain Res. 83 (1997), 117-122). The compound has also been suggested for the treatment of Friedreich's Ataxia and other mitochondrial and neuromuscular diseases (A. O. Hausse, Y. Aggoun, D. Bonnet, D. Sidi, A. Munnich, A. Rotig, P. Rustin, Heart 87 (2002), 346-349).

As a lipophilic compound idebenone is well absorbed in the gastrointestinal tract after conventional oral administration, which is the normal route for administering said compound. Dosage forms such as tablets or capsules have been used in clinical trials and as marketed product. Idebenone fast dissolving tablets are also described in EP 0 553 777 A2. In the course of our investigations on the pharmacological profile of idebenone, we discovered that the compound, after being absorbed in the gut, is metabolized very quickly during its first passage through the liver ("first-pass-effect"). Experiments showed that more than 98% of the idebenone is metabolized during its first passage through the liver. Hepatic metabolism of idebenone results in side chain oxidation, reduction of the quinone ring, sulphate and glucuronide conjugation and subsequent renal excretion. The high first-pass-effect leads to a fast reduction of pharmacologically active plasma levels of free idebenone. Because of this strong first pass metabolism, oral administration of idebenone requires high doses of the compound to achieve pharmacologically efficacious plasma levels in the body. Said high doses can result in unwanted side effects such as diarrhea.

In addition, the requirement for oral formulations of idebenone to be swallowed inflicts difficulties in the practical administration to patients with swallowing problems, e.g. a patient with a serious disease such as Duchenne Muscular Dystrophy or Friedreich's Ataxia, elderly or young patients.

JP-11116470 describes a dermal administration form of idebenone to treat dementia, especially senile dementia of the Alzheimer type. The dermal administration preparation is preferably administered in the form of a cataplasm, a patch or a tape.

However, the dermal administration route has strong limitations regarding an administrable dose for systemic use, patient compliance and dosing accuracy.

The present invention has been accomplished to overcome the above-mentioned disadvantages of the state of the art. Thus, it is the object of the present invention to provide a mode of administering idebenone to a subject in need thereof that avoids the strong first pass metabolism observed after conventional oral administration and thus allows to avoid undesired side effects caused by high doses of said active agent.

It is a further object to facilitate the administration of idebenone for human beings with swallowing problems.

### Summary of the invention

Said object has been achieved by the use of idebenone in the preparation of a medicament for transmucosal administration.

The present invention is limited to the subject-matter as defined in the appended claims. The following description is subject to this limitation.

### Brief Description of the Drawing

Figure 1 shows the plasma levels of idebenone after oromucosal administration (4 mg/kg sublingual (s.l.)) vs. oral (40 mg/kg per os (p.o.)) administration of idebenone in the same Beagle dogs (n=3).
Figure 2 shows the plasma levels of total conjugated (i.e. inactive) metabolites after oromucosal (4 mg/kg s.l.) vs. oral (40 mg/kg p.o.) administration of idebenone in dogs.

### Detailed description of the invention

The present invention relates to the use of idebenone (International Nonproprietary Name (INN): idebenone; Chemical name: 2-(10-Hydroxydecyl)-5,6-dimethoxy-3-methyl-2,5-cyclohexadiene-1,4-dione; Chemical Abstracts Service (CAS) registry number: 58186-27-9) for the preparation of a medicament for transmucosal administration to human beings or animals, which can be highly useful in maintaining or restoring health. Transmucosal formulations comprising as an active ingredient idebenone together with additives and excipients conventionally used in transmucosal formulations are further described herein. Moreover, processes for preparing the transmucosal formulations in question are also described.

Idebenone has the following formula:

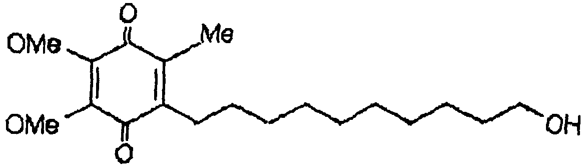

### 2,3-dimethoxy-5-methyl-6-(10-hydroxydecyl)-1,4-benzoquinone, idebenone

Idebenone, a member of the quinone family, has been promoted commercially as a synthetic analog of Coenzyme Q10. Moreover, it has been made the subject of various medical studies investigating its efficacy in the treatment of, for example, neuromuscular diseases such as Friedreich's Ataxia or neurological diseases such as Alzheimer's disease. Idebenone has also been used in topical applications to treat wrinkles. Therefore, idebenone may be regarded as toxicologically safe which means that it can be used as a pharmaceutical active agent in a medicament. The toxicological safety of Idebenone has been confirmed in a clinical study with 536 patients that have been treated with up to 360 mg of idebenone t.i.d. (ter in die). Compared to the placebo treated control group, no treatment emergent adverse events except some gastrointestinal irritations as well as a slight increase in orthopedic events were observed (L. J. Thai, M. Grundman, J. Berg, K. Emstrom, R. Margolin, E. Pfeiffer, M. F. Weiner, E. Zamrini, R. G. Thomas, Neurology 61 (2003), 1498-1502).

It has now been observed that, after conventional oral administration and absorption in the gut, idebenone is rapidly metabolized during its first passage through the liver. The major metabolites are idebenone conjugates such as glucuronates and sulphates as well as derivatives where the side chain of the parent compound has been oxidized. The metabolites of idebenone are pharmacologically not significantly active and they are rapidly excreted. Due to this strong first pass metabolism, oral administration of idebenone requires high doses in order to reach pharmacologically active plasma levels. These high doses result in unwanted effects such as diarrhea and gastrointestinal (GI) tract disturbances which are frequently observed in clinical applications.

It has surprisingly been found that high plasma levels of free idebenone, meaning idebenone which is neither conjugated nor otherwise metabolized, can be reached by *transmucosal administration* of idebenone whereby idebenone can be absorbed into the body through mucosal absorption. Thus, although transmucosal administration of Coenzyme Q10 has been described in an earlier publication (K. Fujii, T. Kawabe, K. Hosoe, T. Hidaka, EP1388340 A1) we unexpectedly were able to demonstrate that the much more polar compound idebenone is rapidly absorbed through mucous membranes. By use of transmucosal formulations the high first pass metabolism observed after conventional oral administration of idebenone can be circumvented.

Circumvention of the strong first pass metabolism of idebenone allows reaching similarly high plasma levels of this drug to be obtained whilst significantly reducing the dose that has to be administered. Lower drug exposure is generally believed to be associated with a reduced risk of adverse side effects and offers a medical advantage leading to improved compliance by the patient.

In addition to circumventing the first pass effect, the medicament according to the invention is effective and easy to handle and therefore has an advantage in terms of practical administration, especially to patients with swallowing difficulties.

In the present invention, "formulations for transmucosal administration" or "transmucosal formulations" means formulations that are in a form intended to be absorbed into the body through the mucosae. According to the invention, such formulations constitute the basis of medicament containing idebenone for transmucosal administration.

Furthermore, according to the present invention, the term "mucosae" includes the nasal mucosa, the colon mucosa and the oral mucosae. The oral mucosae comprise the sublingual and buccal mucosa. Idebenone is preferably applied through the oral or nasal mucosa, more preferably via the sublingual or buccal mucosa.

The medicament for transmucosal administration of idebenone can be prepared, for example, as fast-dissolving tablets, emulsions, *solutions,* sprays, gels, mucoadhesive tablets or pastes, pastilles, sublingual tablets, drops, chewing tablets, suppositories or gums.

Each of these application forms can be produced by conventionally known formulation methods using formulation additives generally used for the production of such formulations. Such additives used for formulations described in the present invention may contain adjuvants or expedients etc. including solvents, buffers, flavoring agents, sweetening agents, fillers, preserving agents, gelling agents, carriers, diluents, *surfactants* and mucoadhesive polymers.

Preferred solvents which can be used in the medicament according to the present invention are alcohols, especially ethanol, fatty acid esters, triglycerides, water and mixtures thereof.

Preferred preserving agents are lower alkyl parahydroxybenzoates, especially methyl and propyl parahydroxybenzoates.

Examples of suitable carriers and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil.

The medicament for transmucosal administration of idebenone according to the present invention can be used in the treatment of various mitochondrial, neuromuscular or neurological diseases. Examples of diseases to be treated include, but are not limited to, Friedreich's Ataxia, Duchenne Muscular Dystrophy, Becker Muscular Dystrophy, Alzheimer's Disease, Leber's Hereditary Optic Neuropathy, MELAS (mitochondrial myopathy, encephalopathy, lactic acidosis with stroke-like episodes), Parkinson's Disease and mitochondrial myopathies.

The effective dosage of idebenone employed may vary depending on e.g. the condition being treated and the severity of the condition being treated. In comparison to the conventional oral route of administration the dosage can be significantly reduced for *the* transmucosal administration without affecting the activity of idebenone. It could be shown that the plasma level of idebenone reaches the required concentration at dosages which have been reduced to a tenth of the dosage in oral application forms.

Suitable doses of idebenone administered by transmucosal formulations are 0.01 mg/kg/day to 60 mg/kg/day. Preferably, idebenone is administered in a dosage of 0.01 mg/kg/day to 20 mg/kg/day, more preferably in a dosage of 0.01 mg/kg/day to 10 mg/kg/day and even more preferably in a dosage of 0.01 mg/kg/day to less than 5 mg/kg/day. Most preferably, the dosage of the active ingredient idebenone is between 0.1 mg/kg/day to 4 mg/kg/day. Studies have shown that, surprisingly, such low dosages achieve the required plasma level of idebenone if it is applied via the oral mucosae. The *required* dosage may be ascertained readily by a person skilled in the art.

In a preferred embodiment, idebenone may be administered in combination with a second therapeutic agent, wherein said second therapeutic agent is preferably selected from glucocorticosteroids such as 6a-methylprednisolone-21 sodium succinate (solumedrol®) or deflazacort (calcort®) which are routinely used in DMD patients for treatment of inflammation and muscle weakness. Likewise, idebenone may be administered in combination with any medicament used in DMD patients to treat DMD-associated cardiomyopathy such as ACE-inhibitors, beta-blockers and diuretics.

In a further preferred embodiment, idebenone may be administered in combination with further therapeutic agents, wherein said further therapeutic agents are preferably erythropoietin, vitamin E, vitamin C, mitoquinone (MitoQ; K. M. Taylor, R. Smith, WO05019232A1), inhibitors of the cysteine protease calpain or inhibitors of the proteasome. Preferred calpain inhibitors are those disclosed in WO 2004/078908 A1, WO 2006/021409 A1 and WO 2006/021413 A1.

Idebenone and the further active agents can be used simultaneously, separately or sequentially in order to treat or prevent the disease symptoms. The active agents may be provided *in* a single dosage form or as separate formulations, each formulation containing at least one of the active agents.

The following examples illustrate the invention, but are not intended to limit the scope of the invention.

### Example 1:

Micro-emulsion formulation.

**Table 1**

| **Ingredient** | **[mg/ml]** | **Function** |
|---|---|---|
| Idebenone | 20 | Active Ingredient |
| Miglyol 812 N | 144 | Solvent |
| TPGS | 96 | Surfactant |
| Water | 750 | Solvent |

Preparation: TPGS (Tocopherol-polyethylenglycol-400-succinate) is preheated to about 60 °C. Miglyol 812 N and idebenone are mixed and sonicated for approx. 10 minutes until the substance is completely dissolved (clear orange solution). The molten TPGS is added to the Miglyol/idebenone solution and stirred. The mixture is diluted and homogenized until a homogeneous emulsion is obtained.

### Example 2:

### Sublingual tablet

**Table 2**

| **Ingredient** | **[mg/tablet]** | **Function** |
|---|---|---|
| Idebenone | 10 | Active Ingredient |
| Lactose monohydrate | 60 | Filler |
| Povidone K30 | 4,5 | Binder |
| Croscarmellose | 5 | Disintegrant |
| Microcrystalline cellulose | 30 | Filler |
| Flavour | 2.5 | Flavouring agent |
| Aspartame | 6 | Sweetener |
| Magnesium stearate | 0.75 | Glidant |
| Talc | 1.25 | Glidant |

Preparation: Idebenone, lactose monohydrate, flavor and aspartame are mixed in a high-shear mixer until a homogeneous mixture is obtained. Povidone is dissolved in water (approx. 8 - 10% solution) and added to the dry mixture and granulated. The wet granules are dried in a fluid bed dryer, sieved and added to a pre-mixture of microcrystalline cellulose, magnesium stearate and talc. The final mixture is compressed to tablets.

### Example 3:

### Spray formulation

**Table 3**

| **Ingredient** | **amount** | **Function** |
|---|---|---|
| Idebenone | 10 g | Active |
| Methylparahydroxybenzoate | 2 g | Preservative |
| Propylparahydroxybenzoate | 0.2 g | Preservative |
| Aspartame | 0.6 g | Sweetener |
| Flavour | 0.6 g | Flavouring agent |
| Ethanol (96%) | 500 ml | Solvent |
| Purified water | q.s. | Solvent |

Preparation: 10 g Idebenone is dissolved in 500 ml ethanol (96%) and is mixed with 400 ml of purified water. Methylparahydroxy benzoate, propylparahydroxy benzoate and aspartame are dissolved in the mixture. The final volume is adjusted to 1000 ml with purified water. The solution is filtered and filled into an appropriate spray device.

### Example 4:

### Sublingual tablet with Idebenone + Prednisone (17-hydroxy-17-(2-hydroxyacetyl)-10,13-dimethyl-7,8,9,10,12,13,14,15,16,17-decahydro-6H-cyclopenta[a]phenanthrene-3,11-dione; CAS 53-03-2)

**Table 4**

| **Ingredient** | **[mg/tablet]** | **Function** |
|---|---|---|
| Idebenone | 10 | Active Ingredient |
| Prednisone | 2.5 | Active Ingredient |
| Lactose monohydrate | 57.5 | Filler |
| Povidone K30 | 4.5 | Binder |
| Croscarmellose | 5 | Disintegrant |
| Microcrystalline cellulose | 30 | Filler |
| Flavor | 2.5 | Flavouring agent |
| Aspartame | 6 | Sweetener |
| Magnesium stearate | 0.75 | Glidant |
| Talc | 1.25 | Glidant |

Preparation: Idebenone, Prednisone, Lactose monohydrate, Flavor and Aspartame are mixed in a high-shear mixer until a homogeneous mixture is obtained. Povidone is dissolved in water (approx. 8 - 10% solution) and added to the dry mixture and granulated. The wet granules are dried in a fluid bed dryer, sieved and added to a pre-mixture of Microcrystalline Cellulose, Magnesium Stearate and Talc. The final mixture is compressed to round shaped tablets.

### Example 5:

### Analytical quantification of idebenone in plasma

Quantification of idebenone was performed by liquid chromatography (LC) coupled on-line with tandem mass spectrometry (MS/MS). Calibration samples were prepared in human plasma and quality control (QC) samples were prepared in dog plasma.

### Sample preparation

150 µl of dog plasma were treated with 10 µl of an EDTA solution, prepared by dissolution of 1245 mg EDTA-potassium salt in 33 ml of 1M NH₄OAc, and 300 µl of Methanol. After vigorous shaking, samples were centrifuged for 45 minutes with 1960 RCF at 4 °C. A 50 µl aliquot of the supernatant was injected into the LC-MS/MS system. All unknown, calibration and QC samples were subjected to the same assay procedure.

Samples were stored at approx. 8 °C for maximally 24h if not used immediately.

### Chromatographic conditions

Idebenone was separated and quantified by HPLC-MS/MS: For HPLC, a Synergi™ 4µ MAX-RP (50 x 2 mm) column (Phenomenex, Schlieren, Switzerland) was used. Column temperature: 50 °C. Mobile phase A: water + 30mM NH₄OAc; mobile phase B: MeOH/H₂O 100/3 (v/v) + 30 mM NH₄OAc, gradient elution (table 4). Flow: 250 µl/min and 400µl/min.

Once separated, idebenone was quantified by ESI-MS/MS (API 4000, Perkin-Elmer-Europe BV, Rotkreuz, Switzerland) in positive mode.

**Table 5: Pump-gradient program and time events for separation and quantification of idebenone**

| Time [min] | mobile phase B [%] | Flow [µl/min] | Comments |
|---|---|---|---|
| 0.01 | 50 | 250 | start gradient, HPLC eluent to MS |
| 3.00 | - | 250 | - |
| 3.01 | - | 400 | - |
| 3.75 | 95 | 400 | end gradient |
| 4.50 | 95 | 400 | - |
| 4.51 | 50 | 400 | - |
| 5.90 | 50 | 400 | - |
| 5.91 | 50 | 250 | - |
| 29.99 | 50 | 250 | pump shutdown |
| 30.00 | 95 | 20 | |

From times 0.01 to 3.75 min a linear gradient was used.

Idebenone conjugates such as glucuronates and sulphates have been quantified after acidic hydrolysis as described by R. Artuch, C. Colomé, M. A. Vilaseca, A. Aracil. M. Pineda, J. Neurosci. Meth. 115 (2002), 63-66.

### Example 6:

Pharmacokinetic data after sublingual delivery of idebenone

Plasma levels of idebenone were studied after the administration of a micro-emulsion, prepared as described in example 1, to dogs (n = 3). The sublingual administration of 4 mg/kg was compared with 40 mg/kg oral administration in a cross-over design.

Blood samples were collected for 8 hours after administration. Concentrations of idebenone in plasma were measured by HPLC-MS/MS as described in example 4 and pharmacokinetic parameters were calculated.

The pharmacokinetic analysis included maximum plasma concentration (Cₘₐₓ). the time when maximum plasma concentration was observed (Tₘₐₓ). and the area under the plasma concentrations versus time curve from time 0 h to 480 min (AUC₀₋₄₈₀ₘᵢₙ).The relative bioavailability of idebenone after sublingual administration compared to the oral administration was calculated for each dog from normalized (1 mg/kg) AUC values. The AUC ratios of the metabolites were also calculated. In addition, Cₘₐₓ ratios, normalized to a 1 mg/kg dose, were calculated.

The obtained results are shown in Tables 6 and 7 below.

**Table 6**

| Mean pharmacokinetic parameters of idebenone and total conjugated (i.e. inactive) metabolites after oral (40 mg/kg) vs. sublingual (4 mg/kg) administration in dogs. Numbers in round brackets indicate the minimum and maximum values observed. | | | | |
|---|---|---|---|---|
| Dosing | Parameter | Cₘₐₓ [ng/ml] | Tₘₐₓ [min] | AUC₀₋₄₈₀ [min*ng/ml] |
| 4 mg/kg oromucosal | idebenone | 105.6 (76.9-133.4) | 6.3 (3-8) | 1265 (675-2261) |
| | conjugates | 871 (272-1260) | 50(15-120) | 55053 |
| 40 mg/kg oral | idebenone | 31.9 (15.21-42.04) | 22.7 (8-30) | 1079.3 (655-1692) |
| | conjugates | 6206 (1824-9663) | 25 (15-30) | 446013 (272990-737465) |

As shown in Table 6, the oromucosal formulation of idebenone, prepared according to example 1, clearly gives superior plasma levels of idebenone compared to conventional oral application.

With 1265 min*ng/ml the AUC₀₋₄₈₀ after oromucosal administration of 4 mg/kg of idebenone is higher than the AUC after oral administration, even if idebenone is administered at a 10-fold higher dose (40 mg/kg, AUC₀₋₄₈₀ = 1079.3 min*ng/ml).

Even more, the maximum plasma concentration after oromucosal administration of 4 mg/kg (105.6 ng/ml) exceeds the Cₘₐₓ after application of 40 mg/kg p.o. (31.9 ng/ml).

**Table 7**

| AUC and Cₘₐₓ ratios for idebenone and total conjugated (i.e. inactive) metabolites of oromucosal (4 mg/kg) and oral (40 mg/kg) administration in dogs. The ratios were normalized to a 1 mg/kg dose. | | |
|---|---|---|
| Idebenone (normalized for dose) | AUC ratio | 11.1 |
| | Cₘₐₓ ratio | 36.8 |
| conjugates (normalized for dose) | AUC ratio | 1.4 |
| | Cₘₐₓ ratio | 1.4 |

Table 7 shows the improvement of the bioavailability of idebenone by oromucosal compared to oral administration after dose normalization to 1 mg/kg. The normalized AUC₀₋₄₈₀ obtained with the oromucosal formulation is 11.1 times higher than the normalized AUC₀₋₄₈₀ obtained after oral administration of idebenone. Parallel to the increase of the normalized AUC₀₋₄₈₀ after oromucosal versus oral administration the normalized Cₘₐₓ increases by a factor of 36.8.

On the other hand, the Cₘₐₓ and AUC₀₋₄₈₀ values of the inactive conjugates of idebenone are only slightly increased after oromucosal compared to oral administration (factor 1.4).

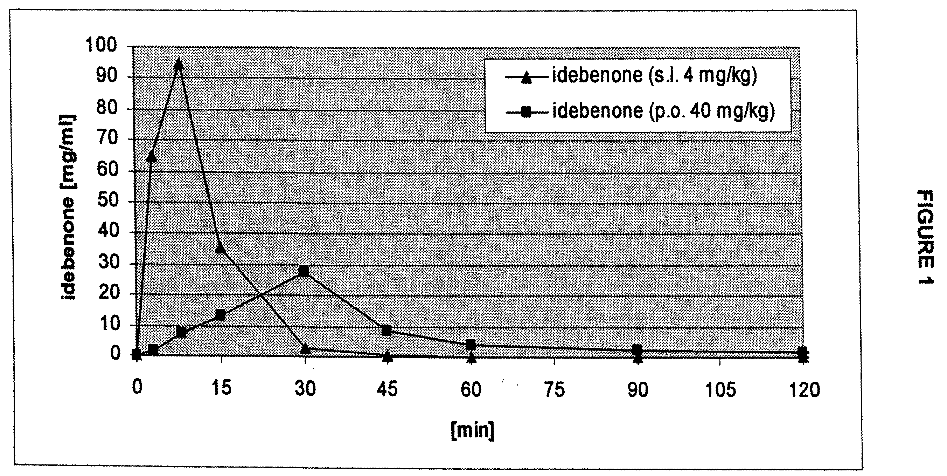

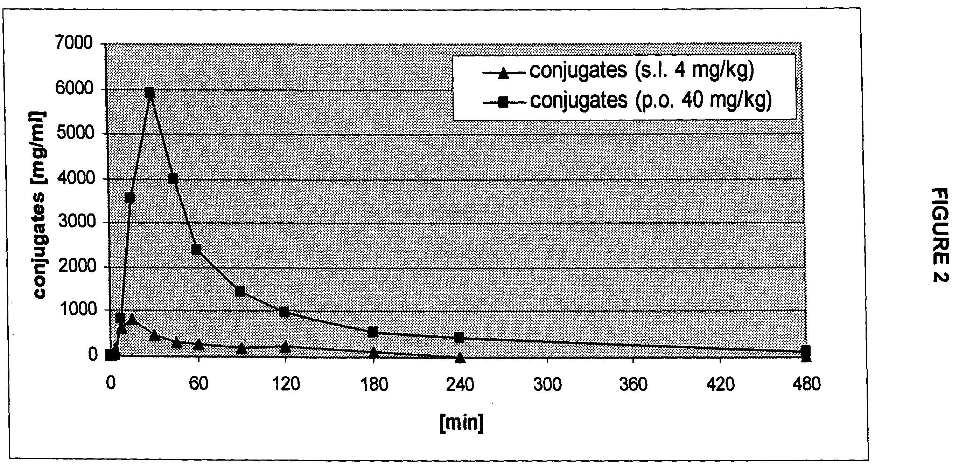

The results are further illustrated in Figures 1 and 2.

Figure 1 shows the mean plasma levels of idebenone after oromucosal administration of 4 mg/kg compared to oral administration of 40 mg/kg administration of idebenone in dogs over the first two hours after administration. The oromucosal formulation results in a much higher Cₘₐₓ and in an earlier Tₘₐₓ

Figure 2 shows the mean plasma levels of the inactive conjugates of idebenone after oromucosal administration of 4 mg/kg compared to oral administration of 40 mg/kg administration of idebenone in dogs over 8 hours after administration.

## Claims

1. Idebenone for use in transmucosal administration, wherein idebenone is administered in a dosage from 0.01 mg/kg/day to less than 5 mg/kg/day.

2. Idebenone according to claim 1 for use in the treatment of mitochondrial, neurological or neuromuscular diseases.

3. Idebenone according to claim 2 for use in the treatment of Friedreich's Ataxia (FRDA), Leber's Hereditary Optic Neuropathy (LHON), mitochondrial myopathy, encephalopathy, lactic acidosis with stroke-like episodes (MELAS) and mitochondrial myopathies.

4. Idebenone according to claim 2 for use in the treatment of Duchenne Muscular Dystrophy (DMD) and Becker Muscular Dystrophy (BMD).

5. Idebenone according to claim 2 for use in the treatment of Alzheimer's disease or Parkinsons's disease.

6. Idebenone according to any of claims 1 to 5, wherein the medicament containing the idebenon is in the form of a suppository, drop, chewing gum, fast dissolving tablet or spray.

7. Idebenone according to any of claims 1 to 6, wherein the medicament containing the idebenone is administered via the nasal mucosa, the oral mucosa or the colon mucosa.

8. Idebenone according to any of claims 1 to 7, wherein the medicament containing the idebenone comprises a second therapeutic agent.

## Patentansprüche

1. Idebenon zur Verwendung in der transmukosalen Verabreichung, wobei Idebenon in einer Dosierung von 0,01 mg/kg/Tag bis zu weniger als 5 mg/kg/Tag verabreicht wird.

2. Idebenon gemäß Anspruch 1 zur Verwendung in der Behandlung von mitochondrialen, neurologischen oder neuromuskulären Krankheiten.

3. Idebenon gemäß Anspruch 2 zur Verwendung in der Behandlung von Friedreichs Ataxie (FRDA), Lebersche hereditäre Optikusneuropathie (LHON), mitochondriale Myopathie, Enzephalopathie, Laktatazidose mit schlaganfallähnlichen Episoden (MELAS) und mitochondrialen Myopathien.

4. Idebenon gemäß Anspruch 2 zur Verwendung in der Behandlung von Muskeldystrophie vom Typ Duchenne (DMD) und Muskeldystrophie vom Typ Becker (BMD).

5. Idebenon gemäß Anspruch 2 zur Verwendung in der Behandlung der Alzheimer-Krankheit oder Parkinson-Krankheit.

6. Idebenon gemäß einem der Ansprüche 1 bis 5, wobei das Medikament enthaltend das Idebenon in der Form eines Zäpfchens, Tropfen, Kaugummi, sich schnell auflösende Tablette oder Spray vorliegt.

7. Idebenon gemäß einem der Ansprüche 1 bis 6, wobei das Medikament enthaltend das Idebenon mittels der Nasenschleimhaut, der Mundschleimhaut oder der Darmschleimhaut verabreicht wird.

8. Idebenon gemäß einem der Ansprüche 1 bis 7, wobei das Medikament enthaltend das Idebenon ein zweites Therapeutikum umfasst.

## Revendications

1. Idébénone à utiliser en administration transmucosique, où l'idébénone est administrée en une dose de 0,01 mg/kg/jour jusqu'à moins de 5 mg/kg/jour.

2. Idébénone selon la revendication 1 à utiliser dans le traitement des maladies mitochondriales, neurologiques ou neuromusculaires.

3. Idébénone selon la revendication 2 à utiliser dans le traitement de l'ataxie de Friedreich (FRDA), de la neuropathie optique héréditaire de Leber (LHON), de la myopathie mitochondriale, de l'encéphalopathie, de l'acidose lactique avec épisodes vasculaires cérébraux (MELAS) et des myopathies mitochondriennes.

4. Idébénone selon la revendication 2 à utiliser dans le traitement de la dystrophie musculaire de Duchenne (DMD) et de la dystrophie musculaire de Becker (BMD).

5. Idébénone selon la revendication 2 à utiliser dans le traitement de la maladie d'Alzheimer ou de la maladie de Parkinson.

6. Idébénone selon l'une quelconque des revendications 1 à 5, où le médicament contenant l'idébénone se présente sous la forme d'un suppositoire, d'une goutte, d'une gomme à mâcher, d'un comprimé à dissolution rapide ou d'une pulvérisation.

7. Idébénone selon l'une quelconque des revendications 1 à 6, où le médicament contenant l'idébénone est administré *via* la muqueuse nasale, la muqueuse buccale ou la muqueuse du côlon.

8. Idébénone selon l'une quelconque des revendications 1 à 7, où le médicament contenant l'idébénone comprend un second agent thérapeutique.
